**Europäisches Patentamt**

**European Patent Office**

**Office européen des brevets**

(19)

(11) Veröffentlichungsnummer: **0 281 047 B1**

## EUROPÄISCHE PATENTSCHRIFT

(12)

(45) Veröffentlichungstag der Patentschrift: **25.09.91**

(51) Int. Cl.5: **A61F 2/18**

(21) Anmeldenummer: **88102973.0**

(22) Anmeldetag: **29.02.88**

(54) **Mittelohr-Prothese.**

(30) Priorität: **06.03.87 DE 3707161**

(43) Veröffentlichungstag der Anmeldung:
**07.09.88 Patentblatt 88/36**

(45) Bekanntmachung des Hinweises auf die
Patenterteilung:
**25.09.91 Patentblatt 91/39**

(84) Benannte Vertragsstaaten:
**AT BE CH DE ES FR GB IT LI NL SE**

(56) Entgegenhaltungen:
**EP-A- 0 231 162**
**GB-A- 1 440 724**
**US-A- 4 169 292**

(73) Patentinhaber: **Fleischer, Gerald, Prof.Dr.**
**Am Rosenberg 31**
**W-6301 Fernwald(DE)**

(72) Erfinder: **Fleischer, Gerald, Prof.Dr.**
**Am Rosenberg 31**
**W-6301 Fernwald(DE)**

(74) Vertreter: **Olbricht, Karl Heinrich, Dipl.-Phys.**
**Am Weinberg 15**
**W-3556 Niederweimar(DE)**

## Beschreibung

Die Erfindung betrifft eine Mittelohr-Prothese gemäß dem Oberbegriff von Anspruch 1

Eine beachtlich große Anzahl von Menschen leidet unter Erkrankungen des Ohres, die von leichter Hörverminderung bis zu schweren Hörschädigungen reichen. Ist das Hörvermögen nur allgemein herabgesetzt, so ist Abhilfe durch Hörgeräte möglich. Bei Schädigung oder Ausfall einzelner Organteile, etwa des Trommelfells oder von Teilen der Gehörknöchelchen, kann man diese durch künstliche Bauelemente ergänzen bzw. ersetzen. Die Operationskunst hat auf diesem Gebiet in den zurückliegenden Jahrzehnten große Fortschritte erzielt.

Beispiele hierfür sind in der US-PS 4 624 672 sowie in der US-PS 4 281 419 angegeben. Hiernach sind Zwischenglieder wie eine Wickelfeder mit mehreren Windungs-Augen oder Gelenkkolben vorgeschlagen worden, die eine mechanische Schwingungs-Verbindung zwischen dem Trommelfell und dem Steigbügel herstellen sollen. Ähnliche Teilprothesen gehen aus der GB-A-1 440 724 und aus der EP-A1-0 231 162 hervor. Anwendbar sind sie jedoch nur, wo eine stützende, gesunde Ohrstruktur dies erlaubt, wo also noch nicht das gesamte Mittelohr geschädigt ist. Leider sind aber gerade solche schweren Erkrankungen recht häufig. Chronische Vereiterungen können mit den herkömmlichen Ersatzmitteln nicht behoben werden.

Das gilt auch für Übertragungs- und Ankopplungs-Elemente gemäß der US-A-4 169 292. Hiernach wird in den (äußeren) Gehörgang ein offener Rohrstutzen eingesetzt, dem ein Abschluß nach außen fehlt. Am inneren Ende des Rohrstutzens befindet sich ein flacher Ring, der einstückig mit einem Hammer ist. Dieser läuft zur Mitte hin in eine schaufelförmige Platte aus, von der ein gewinkelter Amboß absteht, an welchem ein Kolben angreift. Man erkennt, daß mithin lediglich ein künstliches Hebelsystem vorhanden und mit einem in den Gehörgang eingebrachten offenen Rohr verbunden ist.

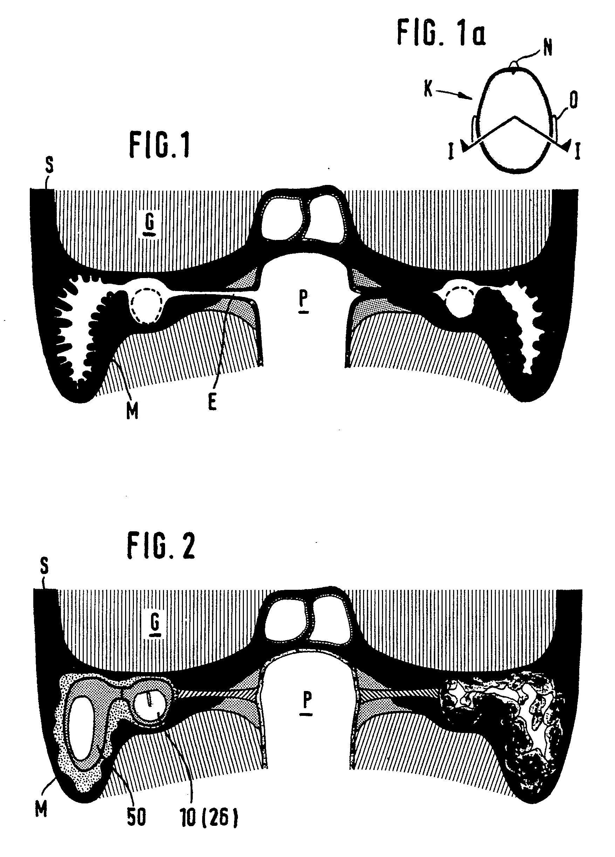

Die volle Funktion des Mittelohrs setzt eine Belüftung der Paukenhöhle voraus. Die in ihr enthaltene Luft wird zum Teil fortlaufend von der Schleimhaut absorbiert, so daß Belüftung und Druckausgleich über die Ohrtrompete notwendig sind. Bei Verschluß der Eustachischen Röhre und bei empfindlicher Schleimhaut kommt es zu wiederholten, später chronischen Erkrankungen des Mittelohres. Es resultiert daraus eine Kette krankhafter Prozesse, die trotz operativer Eingriffe häufig den Verlust des gesamten mechanischen Apparates der Schall-Übertragung ins Innenohr zur Folge hat. Erhalten bleibt der Steigbügel oder nur seine Fußplatte. Als Folge der operativen Beseitigung der Vereiterungen in der knöchernen Wand des Mittelohres - insbesondere im Hohlraumsystem des Mastoids (Fig. 1 und 2) - entsteht die sogenannte Radikalhöhle, d.h. ein operativ erweiterter Mittelohrraum, der von der Außenluft erreicht wird. Trommelfell, Hammer und Amboß fehlen, und die Eustachische Röhre ist verschlossen. Hochgradige Schwerhörigkeit ist die Folge, wobei die bisherigen Operationsmethoden bei diesen schwersten Schädigungen weitgehend versagen.

Insbesondere in diesen, bislang ziemlich hoffnungslosen Fällen soll die Erfindung helfen. Das Innenohr und die Hörbahn sind bei solchen Patienten noch intakt, so daß es möglich ist, eine Mittelohrprothese einzubauen und an das Innenohr anzukoppeln. Weiterhin kann die Prothese dort eingesetzt werden, wo das Mittelohr infolge embryonaler Schädigung nicht ausgebildet, das Innenohr aber entwickelt ist. Schließlich können Unfall-Opfer mit der Prothese ausgerüstet werden, falls Innenohr und Hörbahn noch intakt sind.

Es ist ein wichtiges Ziel der Erfindung, zuverlässige Hilfsmöglichkeiten für solche Patienten zu schaffen, deren Innenohr noch intakt, deren Mittelohr aber weitgehend ausgefallen ist. Auch bei weniger gravierenden Ausfällen soll die Erfindung anwendbar sein, desgleichen bei Haustieren, soweit sie Säugetiere sind.

Zur Lösung dieser Aufgabe sieht die Erfindung eine Mittelohr-Prothese mit den Merkmalen gemäß dem kennzeichnenden Teil von Anspruch 1 vor. Ausgestaltungen sind Gegenstand der Unteransprüche 2 bis 23.

Das Prinzip der Erfindung beruht auf der vollständigen Implantation einer Mittelohr-Totalprothese. Dadurch werden herkömmlich nicht überwindbare Schwierigkeiten überraschend einfach behoben. Insgesamt bildet die Mittelohr-Prothese einen ganz oder nahezu abgeschlossenen Raum mit künstlichen Wandungen; sie hat ein vorgefertigtes Gehäuse, das nach Funktionsprüfung zu Transportzwecken zerlegbar und zum Einbau in Schritten montierbar ist und dann ein komplettes Schallübertragungs-System bildet, gegebenenfalls in Verbindung mit Resten des natürlichen Systems. Im Inneren befindet sich kein lebendes Gewebe. Die vorher meist bestehende chronische Entzündung der Mittelohr-Höhle wird gleichzeitig durch Ausgießen des infizierten Raums mit einer Füllmasse absolut beseitigt. Die Implantation der Prothese führt zur gänzlichen Einbettung und kann so erfolgen, daß Operationsnarben kaum sichtbar sind.

Die neuartige Mittelohr-Prothese hat einen inneren Ankopplungs-Teil der an das Innenohr im Bereich des ovalen Fensters an schließt. Daneben ist ein Abdeck-Teil, der am runden Fenster ein kleines gashaltiges Volumen begrenzt. Nach außen folgt ein Übertragungs-Teil mit künstlichem Trommelfell und einer Verbindung zu einem Mastoid-Teil mit weicher Außenfläche und Dämpfungseinlage. Damit liefert die Prothese nach der Erfindung den vollständigen Apparat für die Schall-Zuführung einschließlich Druckausgleich. Dies überschreitet den Stand der Technik beträchtlich, mit dem nur - soweit brauchbare Verankerungs-Strukturen es erlauben - Einzelteile des schwingenden Systems mehr oder weniger gut ersetzbar sind, so daß die (Wieder-)Herstellung der Hörfähigkeit von Nebenumständen und Zufälligkeiten wie Fingerfertigkeit des operierenden Artzes oder der Art der Einheilung beeinflußt wird.

Der Aufbau des inneren Ankopplungs-Teils ist in Anspruch 2 präzisiert. Wichtig ist der Anschluß an das cvale Fenster mit einem Rohrstutzen, einer Dichtungs-Manschette und einer Abschluß-Membran, die vorzugsweise mit einer als Steigbügel fungierenden zentralen Versteifung als Bestandteil des schwingenden Übertragungs-Systems versehen ist.

In den Ansprüchen 3 bis 13 sind Aufbau, Weiterbildungen und Einzelheiten des Übertragungs-Teils angegeben. Er enthält zwischen dem künstlichen Trommelfell und der Abschluß-Membran einen Impedanz-Wandler, der nach den Ansprüchen 3 bis 6 ein Übertragungsglied oder eine Übertragungskette mit schwingenden, untereinander gegebenenfalls einstellbar gekoppelten und gedämpften Elementen aufweist. Von außen nach innen findet eine Druckverstärkung im Verhältnis 1:7 bis 1:30 oder darüber statt, indem das Schwingungssystem am Trommelfell eine große Krafteingangsfläche hat und mit der kleinen Kraftausgangsfläche der Abschluß-Membran in geeigneter Weise verbunden ist, vorzugsweise über eine Haftfeder nach Anspruch 7.

Wichtig ist die Ausgestaltung laut Anspruch 8, wonach ein äußeres Schwingungssystem auf eine tiefere und ein inneres Schwingungssystem auf eine höhere Hörfrequenz abgestimmt ist, so daß die Hörfläche des Audiogramms jener des gesunden Ohrs durch Überlagerung der beiden Schwerpunkt-Resonanzbereiche weitestgehend nahekommt.

Die Abschlüsse des Übertragungs-Teils nach innen und außen sind in den Ansprüchen 9 bis 13 gekennzeichnet. Besonders bedeutsam sind hierbei die Verankerung und die vollständige Implantation unter Verwendung von biokompatiblen Materialien, so daß die gesamte Prothese im Inneren des Körpers liegt und keine Abstoßungs-Probleme zu gewärtigen sind.

Gemäß Anspruch 14 hat der Mastoid-Teil außen eine weiche Membran. Sie kann laut Anspruch 15 zumindest teilweise luftdurchlässig sein, wenn an dem Schleimhaut-Fenster F eine Belüftung erforderlich ist. Hingegen ist die Rückwand des Mastoid-Teils gemäß Anspruch 16 steifer, jedoch trotz einer gewissen Rückstellkraft ebenfalls nachgiebig zur Anpassung an die bei den einzelnen Menschen stark unterschiedlich gestalteten Raumverhältnisse. Zur Verbesserung der Hörfähigkeit trägt es ferner bei, wenn der Mastoid-Teil gemäß Anspruch 17 mit dem Übertragungs-Teil lösbar und schalldurchlässig verbunden ist. Bevorzugt ist ein Rückschlagventil nach Anspruch 18 und 19 vorhanden, das zur Sicherung gegen Druckstöße und Knallwellen dient.

Zumindest unmittelbar an das Innenohr anschließende Bestandteile können laut Anspruch 20 mit einer Klebermasse verankerbar sein, in die Körperzellen eindringen können, um ein durchwachsenes Gerüst zu bilden. Das Innere der Prothese, jedenfalls das des Übertragungs-Teils, bleibt dabei gewebefrei.

Gemäß Anspruch 21 dienen nachgiebige Laschen zum exakten Zentrieren und Festlegen des Rohrstutzens und/oder der Abschluß-Membran in bezug auf die Fenster des Innenohrs. Nach Anspruch 22 kann ein Verbindungsrohr zum Innenraum des Übertragungs-Teils vorhanden sein.

Eine laut Anspruch 23 abgewandelte Ausführungsform des Ankopplungs-Teils wird dann angewandt, wenn die Steigbügel-Fußplatte des natürlichen Hebelsystems noch intakt ist und nicht entfernt werden soll, um eine Infektionsgefar am Innenohr zu vermeiden.

Weitere Merkmale, Einzelheiten und Vorteile der Erfindung ergeben sich aus dem Wortlaut der Ansprüche sowie aus der folgenden Beschreibung anhand der Zeichnungen. Darin zeigen:

Fig. 1 einen schematisierten Kopf-Schnitt zur Veranschaulichung der Schleimhaut des Mittelohrs, wobei die Schnittebene der Linie I-I in Fig. 1a entspricht,

Fig. 2 einen gleichartigen Kopf-Schnitt,

Fig. 3 eine Ansicht der Lage einer erfindungsgemäßen Prothese in bezug auf die Ohrmuschel,

Fig. 4 eine vergrößerte, schematisierte Schnittansicht des Mittelohr-Aufbaues,

Fig. 5 eine Außenansicht (lateral) einer Mittelohrprothese nach der Erfindung,

Fig. 6 einen Querschnitt durch eine implantierte Mittelohrprothese, wobei die Schnittebene etwa der Linie VI-VI in Fig. 5 entspricht,

Fig. 7 eine stark vergrößerte Schnittansicht eines Klappenventils,

Fig. 8 eine vergrößerte, schematisierte Schnittansicht einer implantierten Mittelohrprothese, wobei die Schnittebene der Linie VIII-VIII in Fig. 5 entspricht,

Fig. 9    eine stark vergrößerte schematisierte Schnittansicht des Ankopplungs-Teils einer Prothese entsprechend dem gestrichelten Bezirk IX in Fig. 8,

Fig. 10    eine gleichfalls stark vergrößerte Draufsicht auf eine Rohrstutzen-Laschen-Anordnung und

Fig. 11    eine Fig. 9 ähnliche Schnittansicht eines abgewandelten Ankopplungs-Teils.

Die Darstellungen der Fig. 1 bis 3 dienen dazu, die Problemstellung zu veranschaulichen. Dabei zeigt Fig. 1a die Draufsicht auf einen Kopf K, wobei zur sicheren Orientierung die Nase N angedeutet ist. Die geknickte Schnittebene I-I führt hinter das Außenohr, das mit den Ohrmuscheln O angedeutet ist.

Man erkennt in Fig. 1 links das gesunde Mittelohr mit dem Mastoid M, wobei der Innenraum über die Eustachische Röhre oder Ohrtrompete E mit dem Naso-Pharynx P in Verbindung steht. Schematisch ist das Gehirn G angedeutet. In der rechten Hälfte von Fig. 1 zeigt sich eine Erkrankung des Mittelohrs durch verdickte Schleimhaut und durch Verengung der Ohrtrompete.

In der rechten Hälfte von Fig. 2 ist der Zustand einer Vereiterung dargestellt, bei dem die Eustachische Röhre bereits vollständig verschlossen ist. Um nun einer Schädigung des Innenohrs vorzubeugen und die Hörfähigkeit (wieder-)herzustellen, wird das gesamte entzündete Gewebe entfernt und die erfindungemäße Mittelohrprothese 10 eingesetzt, wie in Fig. 2 links schematisch angedeutet. Man erkennt, daß von einem Übertragungs-Teil 26 eine Verbindung zu einem Mastoid-Teil 50 vorhanden ist, der sich im Mastoid-Raum M befindet.

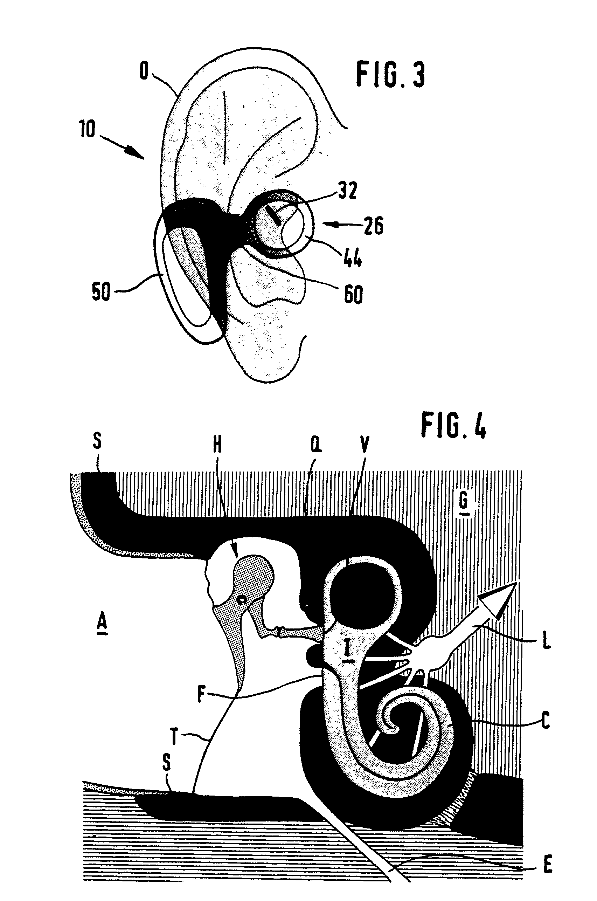

Fig. 3 zeigt die Anordnung der Prothese 10 in bezug auf eine Ohrmuschel O.

Zum besseren Verständnis der Funktion der Prothese 10 ist in Fig. 4 der Mittelohr-Aufbau schematisch dargestellt. An den äußeren Gehörgang A schließt das Trommelfell T an, dessen Rand mit dem Schädelknochen S verwachsen ist. Über ein aus Hammer, Amboß und Steigbügel bestehendes Hebelsystem H wird im äußeren Gehörgang A ankommender Schall unter Druckverstärkung (im statischen Verhältnis 1:22) auf die Steigbügelfußplatte übertragen, die am ovalen Fenster V Schallschwingungen an die Lymphe I des Innenohrs abgibt. Die Membran des runden Fensters F schließt die Schnecke oder Cochlea C des Innenohrs zum Innenraum des Mittelohrs, der Paukenhöhle, hin ab. Der Gehörnerv L führt ins Gehirn G.

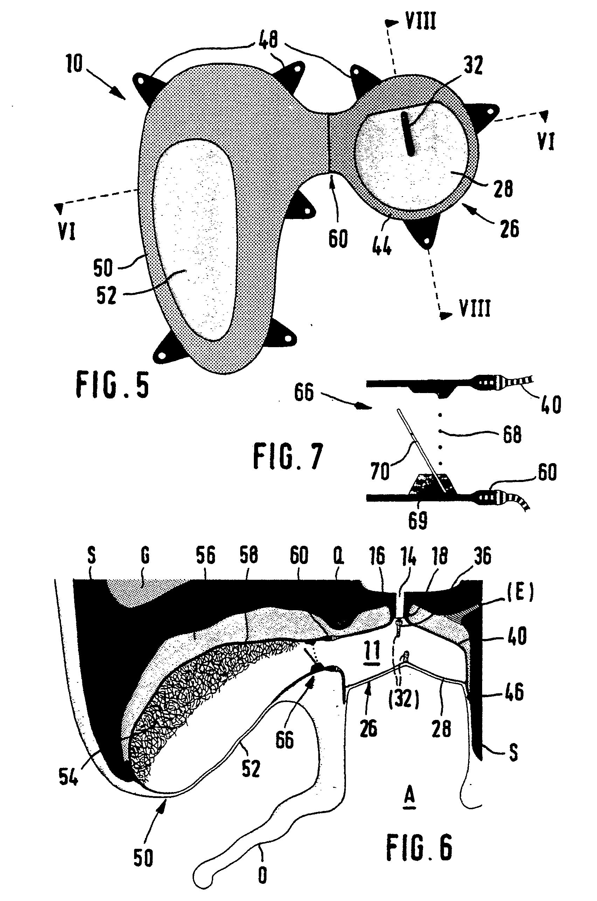

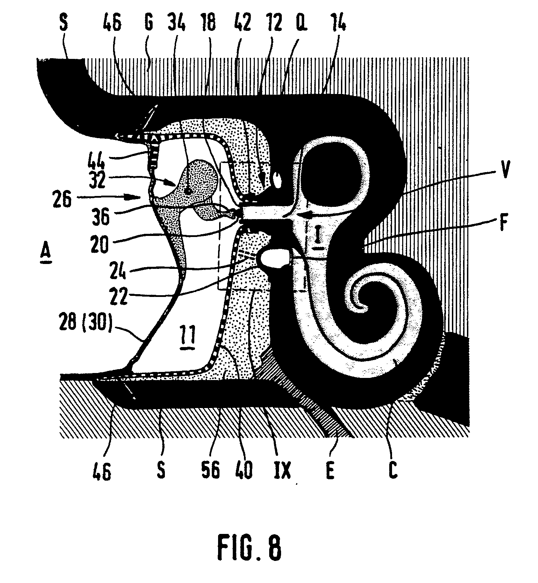

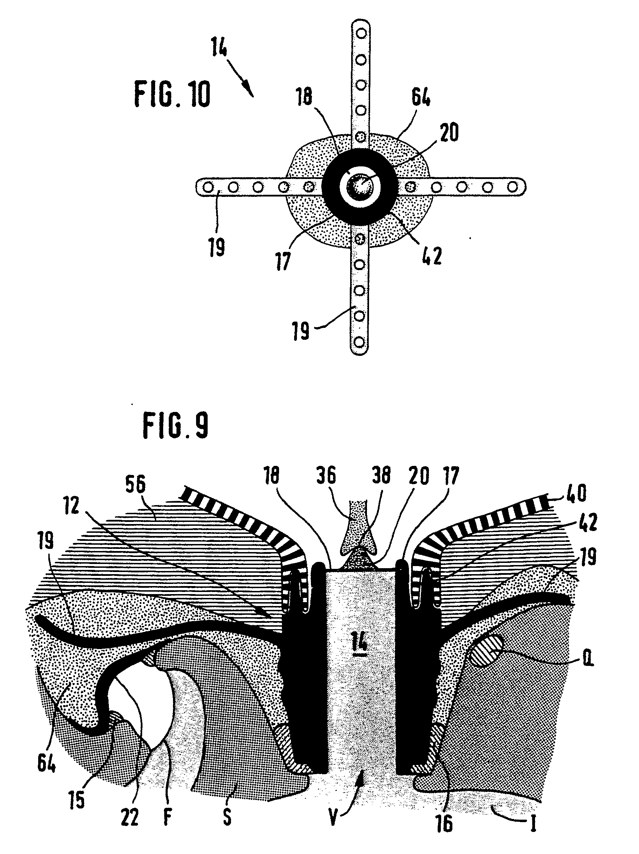

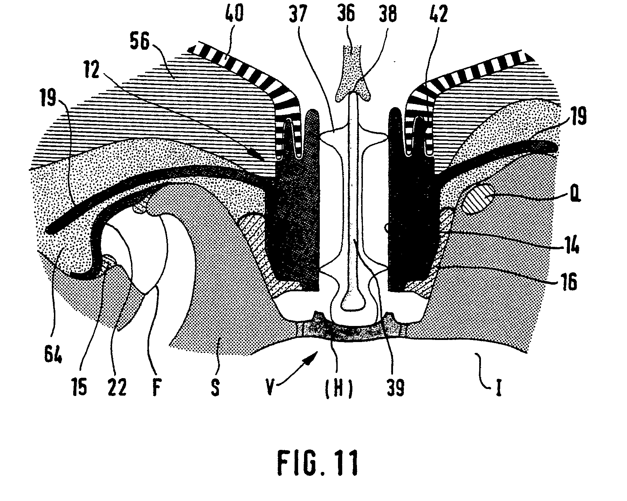

Einen prinzipiell ähnlichen Aufbau hat die Mittelohrprothese 10 nach der Erfindung. Die äußere Anordnung ist aus Fig. 5 ersichtlich, während die einzelnen Bestandteile aus den Fig. 6 bis 11 deutlicher hervorgehen. Die Prothese 10 hat einen Ankopplungs-Teil 12 mit einem Rohrstutzen 14 und einer Dichtungs-Manschette 16, die vorzugsweise aus Polytetrafluorethylen besteht und durch eine mittels eines Rings 17 befestigte Abschluß-Membran 18 verschlossen ist (Fig. 8 und 9). In der Mitte der Abschluß-Membran befindet sich ein künstlicher Steigbügel 20 in Form einer zentralen Versteifung. Zur zentrierten Festlegung dienen insbesondere Laschen 19 (Fig. 10), die mit dem Rohrstutzen 14 einstückig sein und z.B. mittels einer Masse 64 aus Knochenmehl und Fibrinkleber verankert werden können. Das runde Fenster F ist von der Kappe eines Abdeck-Teils 22 überdeckt. Zusätzlich kann, wie in Fig. 8 mit gestrichelten Linien angedeutet ist, ein Verbindungsrohr 24 zum Übertragungs-Teil 26 vorgesehen sein.

Eine Hauptkomponente der Mittelohrprothese 10 ist der Übertragungs-Teil 26. Er hat ein künstliches Trommelfell 28 mit großer Eingangsfläche 30. Von dieser führt ein in Fig. 8 veranschaulichtes Hebelsystem 32 mit einer Drehachse 34 und einer Pfanne 36 über eine kleine Haftfeder 38 zum künstlichen Steigbügel 20 der Abschluß-Membran 18 (vergl. Fig. 9).

Der Innenraum 11 der Prothese 10 ist durch eine Innenwand 40 begrenzt, die über einen vorzugsweise form- und stoffschlüssigen Anschluß 42 mit dem Ankopplungs-Teil 12 verbunden ist. Der Raum zwischen der Innenwand 40 und dem Schädelknochen S sowie den Innenohr- und Ankopplungs-Teilen ist mit einer Füllung 56 aus biokompatiblem Material versehen. Die Eustachische Röhre E ist in diesen Fällen krankheitsbedingt gewöhnlich verschlossen.

Während der Anschluß 42 die dichte Verbindung zum Innenohr hin gewährleistet, ist der Übertragungs-Teil 26 mit dem künstlichen Trommelfell 28 über einen Rahmen 44 außen verankert. Dazu dienen Stifte 46 aus biokompatiblem Material, welche Laschen 48 (Fig. 5) durchsetzen und in den Schädelknochen S eingesetzt sind. Die gesamte Außenfläche ist, wie aus Fig. 6 und 8 ersichtlich ist, mit dünnem Epithel überzogen.

Über eine lösbare Verbindung 60 ist der Übertragungs-Teil 26 mit einem Mastoid-Teil 50 verbunden. Dieser hat zur Ohrmuschel O hin eine weiche Membran 52, die langsamen Druckschwankungen zu folgen vermag und ebenfalls epithelialisiert ist. Zur Anpassung an die bei verschiedenen Patienten recht uanterschiedlichen räumlichen Gegebenheiten ist die Rückwand des Mastoid-Teils 50 etwas nachgiebig, wodurch sich das Einpassen erleichtert. Bei erhöhtem Außendruck wird die weiche Membran 52 nach innen gedrückt, bis innerhalb und außerhalb der Prothese 10 derselbe Druck herrscht. Bei verringertem Außendruck wölbt sich diese weiche Membran 52 automatisch wieder nach außen, bis zum Druck-Ausgleich. Solche langsamen Druck-Schwankungen wirken sich daher praktisch nicht auf das schallübertragende System aus. Zur Sicherung gegen plötzliche, große Druckschwankungen ist am vorzugsweise steckbar ausgebildeten Übergang 60 ein Rückschlagventil 66 vorgesehen. Es hat ein möglichst weitmaschiges Gitter 68 und einseitig eine federnde Klappe 70 (Fig. 7), die vom Mastoid-Teil 50 den Innenraum 11 des Übertragungs-Teils 26 verschließen kann. An die Rückwand 58 schließt eine Dämpfungs-Einlage 54 an, die den Innenraum des Mastoid-Teils 50 teilweise ausfüllt. Dies ist in Fig. 6 veranschaulicht, wo das Hebelsystem 32 des Übertragungs-Teils 26 nur angeschnitten dargestellt und mit gestrichelter Bezugslinie angedeutet ist.

Fig. 11 zeigt eine gegenüber Fig. 9 etwas abgewandelte Ausführungsform eines Ankopplungs-Teils 12, die dann angewandt wird, wenn die Steigbügel-Fußplatte des natürlichen Hebelsystems (H) noch intakt vorhanden ist und nicht entfernt werden soll, da sonst eine zu große Infektionsgefahr für das Innenohr einträte. Diese an einer Ringeinspannung (Lig. annulare) aufgehängte Platte bildet selbst eine Abschluß-Membran, so daß sich eine künstliche Zwischenwand erübrigt. Die Pfanne 36 treibt dann, wiederum am besten über eine Haftfeder 38, einen im Rohrstutzen 14 längsverschieblichen Stößel 39, der mit seinem Fuß kraftschlüssig an der Steigbügel-Fußplatte anliegt und in dem Stutzen 14 durch kolbenartige Kragen 37 geführt, gegebenenfalls auch abgedichtet sein kann.

Die Wandungen der Mittelohrprothese 10 bestehen bevorzugt aus starrem, biokompatiblem Material. Geeignet ist beispielsweise Polytetrafluorethylen oder Titan mit einem Außenüberzug aus biokompatibler Keramik. Einen solchen, allerdings sehr dünnen Verbundaufbau kann auch das künstliche Trommelfell 28 haben. Insbesondere für Ankopplungs-Elemente kommt Polytetrafluorethylen oder ein ähnlicher Werkstoff in Betracht. Die Festlegung kann mit einem Fibrinkleber erfolgen, gegebenenfalls unter Zusatz von körpereigenem Knochenmehl wie für die Haftmasse 64, und zwar sowohl an der Manschette 16 als auch an der mit einem Dichtungsring 15 versehenen Kappe 22. Die schwingenden Elemente im Übertragungs-Teil 26 können durch eine passende Kunststoff-Beschichtung gedämpft werden.

Aufgrund der insgesamt sehr geringen Abmessungen kommt die erfindungsgemäße Prothese 10 mit einer Masse von etwa 0,5 g aus. Durch die Gestaltung des Impedanz-Wandlers im Übertragungs-Teil 26 erreicht man, daß die größte Empfindlichkeit sich auf Frequenzen im Bereich von etwa 1.000 Hz bis 4.000 Hz konzentriert, bevorzugt bei 3.500 Hz.

Die Erfindung ist nicht auf die Anwendung in der Humanmedizin beschränkt; vielmehr kann die Prothese auch bei Tieren eingesetzt werden, namentlich bei Haustieren mit prinzipiell ähnlich aufgebautem Ohr wie Pferden, Hunden, Katzen usw.

| | |
|---|---|
| A | äußerer Gehörgang |
| C | Cochlea |
| E | Eustachische Röhre (Ohrtrompete) |
| F | rundes Fenster mit Membran |
| G | Gehirn |
| H | Hebelsystem: Hammer, Amboß, Steigbügel |
| I | Innenohr-Flüssigkeit(en) |
| K | Kopf |
| L | Gehörnerv |
| M | Mastoid(höhle) |
| N | Nase |
| O | Ohrmuschel |
| P | Naso-Pharynx |
| Q | N. facialis (Mimik-Herv) |
| S | Schädelknochen |
| T | Trommelfell |
| V | ovales Fenster |
| 10 | Mittelohrprothese |
| 11 | Innenraum |
| 12 | Ankopplungs-Teil |
| 14 | Rohrstutzen |
| 15 | Dichtungsring |
| 16 | Dichtungs-Manschette |
| 17 | Ring |
| 18 | Abschluß-Membran |
| 19 | Laschen |
| 20 | künstlicher Steigbügel |
| 22 | Abdeck-Teil bzw. -Kappe |
| 24 | (Verbindungs-)Rohr |
| 26 | Übertragungs-Teil |
| 28 | künstliches Trommelfell |
| 30 | Eingangsfläche |
| 32 | Hebelsystem |
| 34 | Drehachse |
| 36 | Pfanne |
| 37 | Kragen |
| 38 | Haftfeder |
| 39 | Stößel |
| 40 | Innenwand |
| 42 | Anschluß |
| 44 | Rahmen |
| 46 | Stifte |
| 48 | Laschen |
| 50 | Mastoid-Teil |
| 52 | weiche Membran |
| 54 | Dämpfungs-Einlage |
| 56 | Füllung |
| 58 | Rückwand |
| 60 | (Steck-)Verbindung |
| 64 | Fibrinkleber-Haftmasse |
| 66 | Rückschlagventil |
| 68 | Gitter |

69      Haltering
70      Klappe

**Patentansprüche**

1. Mittelohr-Prothese (10) zum Herstellen oder Wiederherstellen der Hörfähigkeit bei Mittelohr-Geschädigten, insbesondere chronisch Erkrankten, die aus folgenden zusammenfügbaren Komponenten eines zum Einbau in Schritten implantierbaren Gehäuses besteht:
a) einem inneren Ankopplungs-Teil (12) zum Anschluß an das ovale Fenster (V),
b) einem vollständigen Schallübertragungs-Teil (26) mit Trommelfell (28) und Impedanzwandler,
c) einem Mastoid-Teil (50) zum Druckausgleich, mit einer Dämpfungseinlage (54).

2. Prothese nach Anspruch 1, dadurch **gekennzeichnet,** daß der Ankopplungs-Teil (12) einen an das ovale Fenster (V) angeschlossenen Rohrstutzen (14) sowie eine DichtungsManschette (16) und eine Abschluß-Membran (18) aufweist, welch letztere mit einem künstlichen Steigbügel (20) in Form einer zentralen Versteifung gekoppelt ist.

3. Prothese nach Anspruch 1 oder 2, dadurch **gekennzeichnet,** daß der zwischen dem Trommelfell (28) und der Abschluß-Membran (18) angeordnete Impedanz-Wandler ein Übertragungsglied oder eine Übertragungskette mit wenigstens einem schwingenden Element (28 bis 36, 20) aufweist, das bevorzugt mit einem Dämpfungsbelag versehen ist.

4. Prothese nach Anspruch 3, dadurch **gekennzeichnet,** daß die Glieder der Übertragungskette (28 bis 36, 20) untereinander mit einstellbarer Kopplung verbunden sind.

5. Prothese nach einem der Ansprüche 1 bis 4, dadurch **gekennzeichnet,** daß der Impedanz-Wandler von außen nach innen eine Druckverstärkung im Verhältnis 1:7 bis 1:30 oder darüber ermöglicht.

6. Prothese nach einem der Ansprüche 3 bis 5, dadurch **gekennzeichnet,** daß die Übertragungskette (28 bis 36, 20) ein System gekoppelter Schwinger aufweist, das am Trommelfell (28) eine große Krafteingangsfläche (30) hat und mit der kleinen Kraftausgangsfläche der AbschlußMembran (18) stoffschlüssig oder formschlüssig mit ständigem Kraftschluß verbunden ist.

7. Prothese nach Anspruch 6, dadurch **gekennzeichnet,** daß als Verbindungselement mit der Abschluß-Membran (18) eine Haftfeder (38) vorhanden ist.

8. Prothese nach einem der Ansprüche 3 bis 7, dadurch **gekennzeichnet,** daß ein äußeres Schwingungssystem (28 bis 32) auf eine tiefere und ein inneres Schwingungssystem (20, 18) auf eine höhere Hörfrequenz abgestimmt ist.

9. Prothese nach wenigstens einem der Ansprüche 1 bis 8, dadurch **gekennzeichnet,** daß der Übertragungs-Teil (26) eine starre Innenwand (40) mit einem lösbar angeordneten, abgedichteten Anschluß (42) an den Ankopplungs-Teil (12) aufweist.

10. Prothese nach wenigstens einem der Ansprüche 1 bis 9, dadurch **gekennzeichnet,** daß der Übertragungs-Teil (26) zumindest mit dem Rand des Trommelfells (28) am bzw. im benachbarten Knochen (S) namentlich mittels Laschen (48) und Stiften (46) verankerbar ist.

11. Prothese nach wenigstens einem der Ansprüche 1 bis 10, dadurch **gekennzeichnet,** daß der Übertragungs-Teil (26) einen ringförmigen Rahmen (44) aufweist, der das Trommelfell (28) haltert.

12. Prothese wenigstens nach Anspruch 11, dadurch **gekennzeichnet,** daß zumindest die das Trommelfell (28) bildende Außenwand und eine Druckausgleichsfläche (52) epithelialisierbar sind.

13. Prothese nach wenigstens einem der Ansprüche 1 bis 12, dadurch **gekennzeichnet,** daß das Trommelfell (28) eine flache, nach innen gerichtete Konus-Membran ist.

14. Prothese nach wenigstens einem der Ansprüche 1 bis 13, dadurch **gekennzeichnet,** daß der MastoidTeil (50) außen eine weiche Membran (52) hat.

15. Prothese nach Anspruch 14, dadurch **gekennzeichnet,** daß die weiche Membran (52) zumindest teilweise luftdurchlässig ist.

16. Prothese nach Anspruch 14 oder 15, dadurch **gekennzeichnet,** daß der Mastoid-Teil (50) eine steifnachgiebige Rückwand (58) aufweist.

17. Prothese nach einem der Ansprüche 1 bis 16, dadurch **gekennzeichnet,** daß der Mastoid-Teil (50) über eine lösbare Verbindung (60) schalldurchlässig mit dem Übertragungs-Teil (26) verbunden ist.

18. Prothese nach Anspruch 17, dadurch **gekennzeichnet,** daß zwischen Mastoid-Teil (50) und ÜbertragungsTeil (26) ein Rückschlagventil (66) vorhanden ist.

19. Prothese nach Anspruch 18, dadurch **gekennzeichnet,** daß das Rückschlagventil (66) an der lösbaren Ver bindung (60) angeordnet und als mit einem Fanggitter (68) versehenes Klappenventil ausgebildet ist.

20. Prothese nach wenigstens einem der Ansprüche 2 bis 19, dadurch **gekennzeichnet, daß** zumindest die Dichtungs-Manschette (16) und ein vor dem runden Fenster (F) aufgesetzter Abdeckteil (22) mittels einer Körperzellen aufnehmenden Klebermasse (64) am Innenohr verankerbar sind.

21. Prothese nach wenigstens einem der Ansprüche 2 bis 20, dadurch **gekennzeichnet, daß** der Rohrstutzen (14) und/oder die Abschluß-Membran (18) mittels nachgiebiger Laschen (19) zentrier- und festlegbar ist bzw. sind.

22. Prothese nach Anspruch 20 oder 21, dadurch **gekennzeichnet,** daß im Abdeck-Teil (22) ein Rohr (24) zur Verbindung mit dem Innenraum (11) des ÜbertragungsTeils (26) vorhanden ist.

23. Prothese nach Anspruch 21 oder 22, dadurch **gekennzeichnet,** daß in dem Rohrstutzen (14) ein Stößel (39) längsverschieblich angeordnet ist, der mit seinem Fuß kraftschlüssig an der Steigbügel-Fußplatte anliegt und in dem Rohr-Stutzen (14) durch kolbenartige Kragen (38) geführt und/oder abgedichtet ist (Fig. 11).

## Claims

1. Middle ear prosthesis (10) for providing or restoring hearing capability of patients affected by middle ear disease, in particular up to chronicity, said prosthesis (10) comprising the following components adapted to be assembled to form a stepwise implanted casing:
   a) an inner coupling unit (12) for connection to the oval window (V),
   b) a complete acoustical transfer unit (26) including a tympanic membrane (28) and an impedance converter,
   c) a mastoid unit (50) for pressure balancing, including a damping insert (54).

2. Prosthesis according to claim 1, wherein the coupling unit (12) comprises a pipe socket (14) connected to the oval window (V) as well as a sealing sleeve (16) and a diaphragm (18) which latter is coupled to artificial stapes (20) by way of central stiffening means.

3. Prosthesis according to claim 1 or claim 2, wherein the impedance converter arranged between the tympanic membrane (28) and the diaphragm (18) comprises a transmission element or a transmission chain including at least one vibratory element (28 to 36, 20) which is preferably coated for attenuation.

4. Prosthesis according to claim 3, wherein the members of the transmission chain (28 to 36, 20) are interconnected by adjustable coupling means.

5. Prosthesis according to any one of claims 1 to 4, wherein the impedance converter is designed for pressure intensification from ambience to the interior (11) at a ratio of 1:7 to 1:30 or above.

6. Prosthesis according to any one of claims 3 to 5, wherein the transmission chain (28 to 36, 20) includes a system of coupled oscillators which system has a large receiving face (30) at the tympanic membrane (28) and is bonded or form-fitted to a small force output face of the diaphragm (18) under permanent physical connection thereto.

7. Prosthesis according to claim 6, wherein an adhesive spring (38) is provided for connection to the diaphragm (18).

8. Prosthesis according to any one of claims 3 to 7, wherein an outer vibratory system (28 to 32) is tuned to a lower frequency and wherein an inner vibratory system (20, 18) is tuned to a higher frequency.

9. Prosthesis according to at least one of claims 1 to 8, wherein the transfer unit (26) includes a rigid inside wall (40) with a detachably arranged sealed connection (42) to the coupling unit (12).

10. Prosthesis according to at least one of claims 1 to 9, wherein the transfer unit (26) is adapted to be secured to or in the adjacent bone (S), at least with the edges of the tympanic membrane (28), in particular by tongues (48) and pins (46).

11. Prosthesis according to at least one of claims 1 to 10, wherein the transfer unit (26) includes an annular frame (44) for holding the tympanic membrane (28).

12. Prosthesis according to claim 11, wherein at least an outer wall that forms the tympanic membrane (28) and a pressure balancing component (52) are adapted to be epithelialized.

13. Prosthesis according to at least one of claims 1 to 12, wherein the tympanic membrane (28) is a flat cone membrane directed inwardly.

14. Prosthesis according to at least one of claims 1 to 13, wherein the mastoid unit (50) includes a soft outer membrane (52).

15. Prosthesis according to claim 14, wherein the soft membrane (52) is at least partly permeable to air.

16. Prosthesis according to claim 14 or claim 15, wherein the mastoid unit (50) includes a resiliently yielding rear wall (58).

17. Prosthesis according to any one of claims 1 to 16, wherein the mastoid unit (50) and the transfer unit (26) are connected by a detachable junction (60) for acoustical transmission.

18. Prosthesis according to claim 17, wherein a check valve (66) is provided between the mastoid unit (50) and the transfer unit (26).

19. Prosthesis according to claim 18, wherein the check valve (66) is adjacent the detachable junction (60) and includes a flap (70) provided with an interceptor grid (68).

20. Prosthesis according to at least one of claims 2 to 19, wherein at least the sealing sleeve (16) and a cover unit (22) affixed in front of the round window (F) are adapted to be secured to the inner ear by means of an adhesive paste (64) for ingrowth of connective tissue cells.

21. Prosthesis according to at least one of claims 2 to 20, wherein the pipe socket (14) and/or the diaphragm (18) is/are adapted to be centered and/or affixed by flexible strap means (19).

22. Prosthesis according to claim 20 or claim 21, wherein the cover unit (22) includes a conduit (24) for connection to the interior (11) of the transfer unit (26).

23. Prosthesis according to claim 21 or claim 22, wherein a plunger (39) is disposed for longitudinal shifting within the pipe socket (14), which plunger has a foot for engaging the stapedial footplate and is guided and/or sealed in the pipe socket (14) by piston-type collars (38).

**Revendications**

1. Prothèse de l'oreille moyenne (10) pour établir ou rétablir la capacité acoustique chez des lésés de l'oreille moyenne, en particulier chez des malades chroniques, qui est composée des éléments assemblables suivants d'un boîtier implantable par étappes pour le montage:
   a) une partie de raccordement interne (12) pour être raccordée à la fenêtre ovale (V),
   b) une partie complète de transmission du son (26) avec un tympan (28) et un transformateur d' adaptation d' impédance,
   c) une partie mastoïde (50) pour la compenser la pression, avec un élément d'amortissement (54).

2. Prothèse conforme à la revendication 1, **caractérisée** par le fait que la partie de raccordement (12) dispose d'une tubulure (14) raccordée à la fenêtre ovale (V), ainsi que d'une garniture d'étanchéité (16) et d'une membrane terminale (18), laquelle est reliée à un étrier artificiel (20) en forme de renforcement central.

3. Prothèse conforme à la revendication 1 ou 2, **caractérisée** par le fait que le transformateur d' adaptation d' impédance situé entre le tympan (28) et la membrane terminale (18) dispose d'un maillon ou d'une chaîne de transmission avec au moins un élément oscillant (28 à 36, 20), qui sera pourvu de préférence d'un revêtement d'amortissement.

4. Prothèse conforme à la revendication 3, **caractérisée** par le fait que les maillons de la chaîne de transmission (28 à 36, 20) soient reliés entre eux par un raccord réglable.

5. Prothèse conforme à l'une des revendications 1 à 4, **caractérisée** par le fait que le transformateur d'adaptation d'impédance permette un renforcement de la pression de l'extérieur vers l'intérieur dans une proportion de 1:7 à 1:30 ou plus.

6. Prothèse conforme à l'une des revendications 3 à 5, **caractérisée** par le fait que la chaîne de transmission (28 à 36, 20) dispose d'un système d'oscillateurs reliés, lequel a une grande surface d'entrée de la force (30) au tympan (28), laquelle est reliée à une petite surface de sortie de la force de la membrane terminale (18) par une matière à engagement positif et avec une transmission constante de la force.

7. Prothèse conforme à la revendication 6, **caractérisée** par le fait qu'il y ait un ressort adhésif (38) servant d'élément de raccordement à la membrane terminale (18).

8. Prothèse conforme à l'une des revendications 3 à 7, **caractérisée** par le fait qu'un système oscillatoire extérieur (28 à 32) soit réglé sur une fréquence acoustique plus basse et qu'un système oscillatoire intérieur (20, 18) le soit sur une fréquence plus haute.

9. Prothèse conforme à au moins une des revendications 1 à 8, **caractérisée** par le fait que la partie de transmission (26) dispose d'un paroi interne rigide (40) avec un raccord (42) sur la partie de raccordement (12), qui est étanché et agencé de façon détachable.

10. Prothèse conforme à au moins une des revendications 1 à 9, **caractérisée** par le fait que la partie de transmission (26) puisse au moins être ancrée avec le bord du tympan (28) sur ou dans l'os voisin (S), notamment par le moyen de languettes (48) et de pointes (46).

11. Prothèse conforme à au moins une des revendications 1 à 10, **caractérisée** par le fait que la partie de transmission (26) dispose d'un cadre en forme d'anneau (44) servant de support au tympan (28).

12. Prothèse conforme au moins à la revendication 11, **caractérisée** par le fait qu'au moins la paroi externe formant le tympan (28) et une surface de compensation de la pression (52) soient épithélialisables.

13. Prothèse conforme à au moins une des revendications 1 à 12, **caractérisée** par le fait que le tympan (28) soit une membrane conique plate dirigéevers l'intérieur.

14. Prothèse conforme à au moins une des revendications 1 à 13, **caractérisée** par le fait que la partie mastoïde (50) ait une membrane douce (52) à l'extérieur.

15. Prothèse conforme à la revendication 14, caractérisée par le fait que la membrane douce (52) soit au moins partiellement perméable à l'air.

16. Prothèse conforme à la revendication 14 ou 15, **caractérisée** par le fait que la partie mastoïde (50) dispose d'un paroi arrière (58) rigide mais souple.

17. Prothèse conforme à l'une des revendications 1 à 16, **caractérisée** par le fait que la partie mastoïde (50) soit reliée par un raccord détachable (60) à la partie de transmission (26) de manière à laisser passser le son.

18. Prothèse conforme à la revendication 17, **caractérisée** par le fait qu'il y ait une soupape à clapet (66) entre la partie mastoïde (50) et la partie de transmission (26).

19. Prothèse conforme à la revendication 18, **caractérisée** par le fait que la soupape à clapet (66) soit agencée sur le raccord détachable (60) et qu'elle soit munie d'une grille d'arrêt (68).

20. Prothèse conforme à au moins une des revendications 2 à 19, **caractérisée** par le fait qu'au moins la garniture d'étanchéité (16) et une partie de recouvrement (22) située devant la fenêtre ronde (F), soient ancrables sur l'oreille interne au moyen d'une masse adhésive (64) acceptant les cellules du corps.

21. Prothèse conforme à au moins une des revendications 2 à 20, **caractérisée** par le fait que la tubulure (14) et/ou la membrane terminale (18) puisse(nt) être centrée(s) et fixée(s) au moyen de languettes flexibles (19).

22. Prothèse conforme à la revendication 20 ou 21, **caractérisée** par le fait q'il y ait dans la partie de recouvrement (22) un tube (24) pour le raccordement avec l'intérieur (11) de la partie de transmission (26).

23. Prothèse conforme à la revendication 21 ou 22, **caractérisée** par le fait qu'une tige (39) soit disposée dans la tubulure (14) de manière à pouvoir coulisser longitudinalement, touchant de son pied la base de l'étrier de manière à être entrainée par la force et étant guidée et/ou étanchée (fig. 11) dans la tubulure (14) par des collets en forme de pistons (38).

FIG. 1a

FIG.1

FIG. 2

FIG. 3

FIG. 4

FIG.5

FIG.7

FIG.6

FIG. 8

FIG. 10

FIG. 9

FIG. 11